# EUROPÄISCHE PATENTSCHRIFT

(11) **EP 2 333 077 B1**
(45) Veröffentlichungstag und Bekanntmachung des Hinweises auf die Patenterteilung: **31.07.2013**
(21) Anmeldenummer: 10012269.6
(22) Anmeldetag: 23.11.2005
(51) Int. Cl.: C12N 15/82, A01H 5/00

(54) **Lagerungsinduzierter Promotor**
Storage-induced promoter
Promoteur induit au stockage

(30) Priorität: 26.11.2004 DE 102004057291
(43) Veröffentlichungstag der Anmeldung: 15.06.2011
(62) Teilanmeldung aus: 05817889.8
(73) Patentinhaber: KWS Saat AG, 37555 Einbeck (DE); SÜDZUCKER AKTIENGESELLSCHAFT, 68165 Mannheim (DE)
(72) Erfinder: Stahl, Dietmar Jürgen, 37574 Einbeck (DE); Hehl, Reinhard, 38114 Braunschweig (DE); Rotthues, Alexander, 61462 Königstein im Taunus (DE)
(74) Vertreter: Pohl, Manfred

(56) Entgegenhaltungen:
- EP-A- 0 812 917
- WO-A-00/31251
- WO-A-02/40687
- WO-A-95/03690
- WO-A2-2004/002216
- TRINDADE LUISA M ET AL: "Analysis of genes differentially expressed during potato tuber life cycle and isolation of their promoter regions.", PLANT SCIENCE (OXFORD), Bd. 166, Nr. 2, Februar 2004 (2004-02), Seiten 423-433, XP007900227, ISSN: 0168-9452
- CHEN LONG-FANG O ET AL: "Transformation of broccoli (Brassica oleracea var. italica) with isopentenyltransferase gene via Agrobacterium tumefaciens for post-harvest yellowing retardation", MOLECULAR BREEDING, Bd. 7, Nr. 3, 2001, Seiten 243-257, XP002462319, ISSN: 1380-3743
- WINICHAYAKUL SOMRUTAI ET AL: "Distinct cis-elements in the Asparagus officinalis asparagine synthetase promoter respond to carbohydrate and senescence signals", FUNCTIONAL PLANT BIOLOGY, Bd. 31, Nr. 6, 23. Juni 2004 (2004-06-23), Seiten 573-582, XP008086819, ISSN: 1445-4408
- REILLY K ET AL: "Isolation and characterisation of a cassava catalase expressed during post-harvest physiological deterioration<1>", BIOCHIMICA ET BIOPHYSICA ACTA . GENE STRUCTURE AND EXPRESSION, ELSEVIER, AMSTERDAM, NL, Bd. 1518, Nr. 3, 16. April 2001 (2001-04-16), Seiten 317-323, XP004235053, ISSN: 0167-4781
- HUANG JIANG ET AL: "Molecular analysis of differentially expressed genes during postharvest deterioration in cassava (Manihot esculenta Crantz) tuberous roots", EUPHYTICA, Bd. 120, Nr. 1, 2001, Seiten 85-93, XP002462320, ISSN: 0014-2336
- PAGE TANIA ET AL: "Molecular and biochemical characterization of postharvest senescence in broccoli", PLANT PHYSIOLOGY (ROCKVILLE), Bd. 125, Nr. 2, Februar 2001 (2001-02), Seiten 718-727, XP002462321, ISSN: 0032-0889

## Beschreibung

Die vorliegende Erfindung betrifft einen Promotor mit einer organspezifischen Aktivität in Pflanzen, dessen Verwendung sowie transgene Pflanzen.

Mit Blick auf die Lagerfähigkeit können Nutzpflanzen gemäß Nilsson (2000) in drei Gruppen eingeteilt werden. Das Erntegut der ersten Gruppe, zu der Pflanzen wie Kohl, Broccoli, Blumenkohl, Spargel und Spinat gehören, umfasst Blätter, Sprosse, Blüten und Knospen. Die Pflanzenteile dieser Pflanzen haben geringe Wasserspeicherfähigkeiten und zeigen schnell eine nacherntliche Seneszenz. Zu der zweiten Gruppe gehören Pflanzen mit fleischigen Früchten, wie z.B. Tomate, Kürbis und Birnen. Die Früchte dieser Pflanzen zeigen eine Reifung und Seneszens während der Lagerung. Zu der dritten Gruppe von Pflanzen zählen zweijährige Pflanzen. Pflanzen mit einem zweijährigen Lebenszyklus, wie Zuckerrübe, Zicchorie, Karotte, Zwiebeln oder Artischocke entwickeln im Verlauf des ersten Jahres ein Speicherorgan, das im zweiten Jahr zur Blüte und Samenbildung beiträgt.

Speicherorgane unterliegen nach der Ernte (postharvest) zahlreichen physiologischen Veränderungen, die die Qualität der Speicherorgane und die Quantität ihrer Inhaltsstoffe beeinflussen. Die physiologischen Veränderungen sind zum einen die Folgen der mechanischen Behandlung während des Rodens, wie Verwundungen und Quetschungen und zum anderen die Auswirkung der Lagerung und des damit verbundenen Wasserverlustes, die Konsequenzen einer erzwungenen oder natürlichen Dormanz oder das Ergebnis einer Kälteadaptation.

Um Stoffwechselleistungen eines Speicherorgans nach der Ernte beeinflussen zu können, bedarf es daher gezielter Prozesse, die vorzugsweise über spezifische Promotoren reguliert werden. Es sind allerdings Gene bekannt, die im Sproß von Spargel oder den Blüten von Broccoli nach der Ernte aktiviert werden. In Speicherorganen von Pflanzen kennt man dagegen solche Gene nicht, so daß Hinweise auf geeignete Promotoren bislang nicht vorliegen.

Trinidade et al. "Analysis of genes differentially expreseed cluring potato tuber life cycle and isolation of their promoter regions", PLANT SCIENCE (OXFORD), Bd. 166, Nr. 2, Februar 2004 (2004-2), Selten 423-433, XP007900227, ISSN: 0168-9452, weisen auf einzelne Gene hin, die während der Kartoffelknollenbildung exprimiert wurden.

Aus EP 0 812 917 A sind kälteregulierte Promotoren bekennt die keine lagerungsspezifische Aktivität aufweisen.

Darüber hinaus offenbart WO 02/40687 A Promotoren aus Zuckerrübe, die jedoch ebenfalls nicht lagerungsinduziert sind.

Aufgabe der vorliegenden Erfindung ist es daher, einen solchen Promotor bereitzustellen, mit dessen Hilfe stoffwechselphysiologische Veränderungen in Speicherorganen von Pflanzen nach deren Ernte beinflußt werden können.

Erfindungsgemäß erfolgt die Lösung der gestellten Aufgabe durch einen Promotor mit den Merkmalen des Anspruchs 1.

Einige der in dieser Anmeldung verwendeten Begriffe werden zunächst näher erläutert:
Im Sinne dieser Erfindung sind Speicherorgane einer Pflanze solche Organe, die der Speicherung von Kohlenhydraten wie Sucrose, Stärke oder Inulin und/oder von Stickstoffverbindungen wie Proteinen oder Aminosäuren dienen. Ein typisches Speicherorgan ist zum Beispiel die Wurzel oder das Hypokotyl. Sproßartige Speicherorgane können Knollen der Kartoffel, des Topinamburs und die Halminternodien des Zuckerrohres sein. Weitere Speicherorgane sind Wurzelknollen , wie sie bei Yam, Maniok und Batate vorkommen.

Zweijährige Pflanzen benötigen für ihren Lebenszyklus eine 2-jährige Entwicklungsperiode. In dem 1. Jahr wird von den Pflanzen ein Speicherorgan angelegt. In dem 2. Jahr erfolgt unter Ausnutzung der Reservestoffe des Speicherorgans die Bildung von Blüten und die Samenbildung.

Unter einem Promotor wird eine Nukleinsäuresequenz verstanden, die die Expression eines Gens gegebenenfalls in Abhängigkeit von endogenen und exogenen Faktoren steuert. Zu diesen Faktoren gehören z. B. Induktoren, Repressoren und ähnliche DNAbindende Proteine, aber auch Umwelteinflüsse. Ein Promotor kann aus mehreren Elementen bestehen. Er umfaßt jedoch mindestens ein regulatorisches Element, das für die Transkription des unter seiner Kontrolle stehenden Gens zuständig ist.

Ein Promotor, der in einem Speicherorgan nach der Ernte aktiver ist als vor der Ernte und dessen Aktivität somit induziert wird, zeigt beispielsweise in geernteten Wurzeln eine durch RNA-Blots messbare Aktivität, die bei vergleichbaren Versuchsbedingungen in nichtgeernteten Wurzeln zu weniger als 20%, vorzugsweise zu weniger als 10% und insbesondere zu weniger als 5% nachweisbar ist. Diese Spezifität kann erst mit einiger Verzögerung nach dem Roden und während der Lagerung auftreten.

"Derivate" eines Promotors sind verkürzte oder verlängerte oder abschnittsweise identische Versionen oder Homologe dieses Promotors mit den gleichen oder im wesentlichen gleichen Eigenschaften.

"Direkte antifungale bzw. antibakterielle Wirkung" bedeutet, dass Genprodukte unmittelbar antifungal wirken, indem sie z. B. Zellwände auflösen oder für Phytoalexinsynthasen codieren bzw. für Metabolite, die hemmend in den pilzlichen oder bakteriellen Stoffwechsel eingreifen.

"Indirekte antifungale bzw. antibaktereille Wirkung" bedeutet, dass Genprodukte die pflanzliche Genabwehr aktivieren. Zu diesen Genen gehören z. B. Resistenzgene, Komponenten der Signaltransduktion (wie Kinasen, Phosphatasen), Transkriptionsfaktoren oder Enzyme, die Signalsubstanzen produzieren (wie Ethylen bildende, Salicylsäure bildende oder Jasmonat bildende Enzyme, reaktive Sauerstoffspezies bildende Enzyme, Stickstoffmonoxyd bildende Enzyme).

Unter "Infektion" ist der früheste Zeitpunkt zu verstehen, bei dem der Stoffwechsel des Pilzes (bzw. das Wachstum des Pilzes) auf eine Penetration des Wirtsgewebes vorbereitet wird. Dazu gehören z. B. das Auswachsen von Hyphen oder die Bildung von spezifischen Infektionsstrukturen wie Penetrationshyphen und Appressorien.

Der Ausdruck "Homologie" bedeutet hierbei eine Homologie von mindestens 70 % auf DNA-Ebene, die gemäß bekannter Verfahren, z.B. der computergestützten Sequenzvergleiche (S.F. Altschul et al., 1990), Basic Local Alignment search tool, J.Mol. Biol. 215: 403-410) bestimmt werden kann.

"Komplementäre Nukleotidsequenz" bedeutet bezogen auf eine doppelsträngige DNA, daß der zum ersten DNA Strang komplementäre zweite DNA Strang entsprechend den Basenpaarungsregeln die Nukleotidbasen aufweist, die zu den Basen des ersten Stranges korrespondieren.

Der hier verwendete Begriff "hybridisieren" bedeutet hybridisieren unter üblichen Bedingungen, wie sie in Sambrook et al. (1989) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. "Stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

Die Erfindung wird nachfolgend und mit Bezug auf die Figuren und Beispiele näher erläutert.

Der erfindungsgemäße Promotor ist in den Speicherorganen von Pflanzen, wie der Wurzel der Zuckerrübe, Karotte und Zichorie oder den Knollen der Kartoffel nach der Ernte aktiv. Keine oder eine nur geringe Aktivität ist hingegen in den Speicherorganen oder den anderen Organen vor der Ernte nachweisbar. Diese Eigenschaft kann zur Verbesserung des Stoffwechsels des Speicherorgans nach der Ernte genutzt werden. Unter Verwendung des Promotors lassen sich daher auch transgene Pflanzen und Teile dieser Pflanzen wie Samen herstellen.

In bevorzugter Weise ist die Aktivität des erfindungsgemäßen Promotors in den Speicherorganen durch RNA-Blots messbar, die bei vergleichbaren Versuchsbedingungen vor der Ernte der Speicherorgane zu weniger als 20%, vorzugsweise zu weniger als 10% und insbesondere zu weniger als 5% nachweisbar ist. Auch Derivate eines solchen Promotors können vorgesehen sein. Solche Derivate sind oben näher definiert und umfassen auch DNA-Fragmente des Promotors, wie sie in den Resriktionskarten wiedergegeben sind, sowie DNA-Fragmente, die durch Einsatz nicht ausdrücklich genannter handelsüblicher Restriktionsendonukleasen erhältlich sind.

Die Erfindung betrifft sodann transgene Pflanzen, die mit dem erfindungsgemäßen Promotor transformiert wurden.

Die Erfindung betrifft ebenfalls die Verwendung des erfindungsgemäßen Promotors oder von Derivaten zur Herstellung einer transgenen Pflanze mit einer oder mehreren der folgenden Eigenschaften:
a) Verbessertem Kohlenhydratmetabolismus in Speicherorganen nach der Ernte
b) Verbessertem Stickstoffmetabolismus in Speicherorganen nach der Ernte
c) Verbesserter Trockenstressresistenz und verbessertem Wasserstatus in Speicherorganen nach der Ernte
d) Verbesserter Kälte- und Frosttoleranz von Speicherorganen nach der Ernte
e) Erhöhter Resistenz/Toleranz von Speicherorganen gegenüber Pathogenen nach der Ernte
f) Verbessertem Sekundärstoffwechsel in Speicherorganen nach der Ernte

Der erfindungsgemäße Promotor kann daher genutzt werden, um den Abbau von Zucker in der geernteten Zuckerrübe und die Akkumulation von Invertzucker zu reduzieren bzw. zu verhindern. Dazu wird z.B. ein Invertaseinhibitorgen in der geernteten Wurzel exprimiert und die Aktivität von zellulären Invertasen gehemmt.

Der erfindungsgemäße Promotor kann auch genutzt werden, um höhere Ausbeuten an Inulin und die Produktion von längerkettigeren Inulinmolekülen in der Zichorienwurzel zu erreichen. Dazu wird der Abbau von Inulin in der geernteten Zichorie reduziert bzw. verhindert, indem die Aktivität der Fructosyl-Exohydrolase in der Wurzel nach der Ernte durch einen Antisense- oder RNAᵢ-Ansatz reduziert wird.

Der erfindungsgemäße Promotor kann ebenfalls genutzt werden, um das "coldsweetening" der geernteten und gelagerten Kartoffelknollen zu reduzieren. Dazu wird ein Invertaseinhibitorgen in der geernteten Knolle exprimiert und die Aktivität von zellulären Invertasen gehemmt.

Der erfindungsgemäße Promotor kann weiterhin genutzt werden, um den Gehalt von extrahierbarem "schädlichen Stickstoff', wie Aminosäuren, in den Speicherorganen der Pflanze nach der Ernte zu reduzieren. Erhöhte Konzentrationen an N-Verbindungen in Speicherorganen reduzieren oft den ernährungsphysiologischen Wert von Ernteprodukten oder erschweren die Isolierung von Speicherstoffen wie Sucrose aus Zuckerrübenwurzeln. Eine Reduktion von extrahierbarem "schädlichen Stickstoff' in der Wurzel kann durch einen verstärkten Einbau von Aminosäuren in Proteine in die Speicherorgane mit Hilfe der Promotoren erreicht werden. Proteine können im Gegensatz zu Aminosäuren während der Zuckergewinnung aus der Zuckerrübe gefällt werden und sind daher nicht extrahierbar.

Der erfindungsgemäße Promotor kann auch genutzt werden, um die Kälte- und Frosttoleranz der Speicherorgane zu verbessern. Dazu werden z.B. Transkriptionsfaktoren für Kälte- oder Frosttoleranz oder Kälte- oder Frostschutzproteine durch den Promotor exprimiert.

Mit Hilfe des erfindungsgemäßen Promotors kann auch die Krankheitsresistenz der geernteten Speicherorgane verbessert werden. Zahlreiche bodenbürtige Pilze, wie Vertreter der Spezies *Fusarium* spp., oder Bakterien, wie *Erwinia carotovora*, der Erreger der Kartoffelknollennassfäule, können geerntete Speicherorgane infizieren. Durch Kombination des induzierbaren Promotors mit einem Gen, dessen Genprodukt eine direkte oder indirekte antifungale oder antibakterielle Wirkung ausübt, kann eine Pilz- oder Bakterienresistenz verwirklicht und die Lagerfähigkeit der Speicherorgane verbessert werden.

Der erfindungsgemäße Promotor kann schließlich genutzt werden, um den Sekundärstoffwechsel der Speicherorgane zu verbessern. Kartoffelknollen sind die wichtigste Vitamin C Quelle in Mitteleuropa. Der Vitamin C-Gehalt der Knollen nimmt jedoch während der Lagerung ab. Mit Hilfe der Promotoren kann dieser Vitaminabbau verhindert bzw. reduziert werden.

### Abbildungen

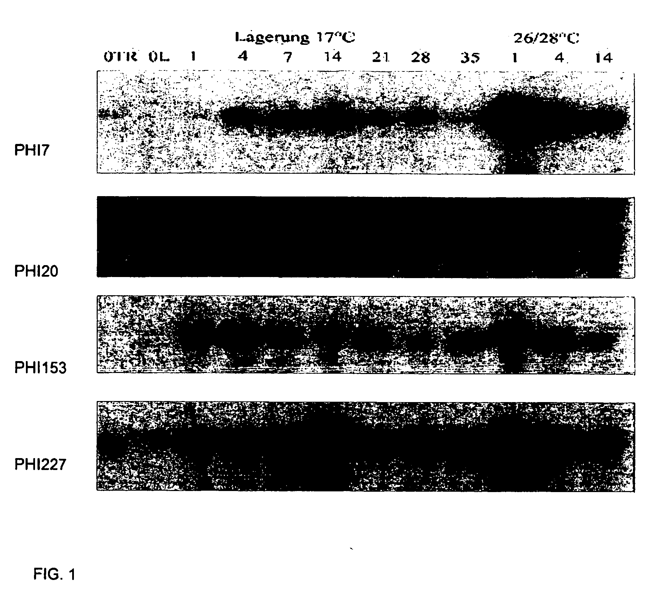

Abbildung 1 zeigt die Induktion der PHI (postharvest induced) -Gene 7, 20, 153 und 227 der Zuckerrübe nach der Ernte der Rübenwurzeln durch ein RNA-Blot Experiment. Analysiert wurde die Genexpression in Blättern und Wurzeln der Zuckerrübe unmittelbar vor der Ernte (0d) und dann zu verschiedenen Zeitpunkten nach der Ernte. Dazu wurden die Wurzeln 1, 4, 7, 14, 21, 28 und 35 Tage bei 17 °C bzw. 1, 4 und 14 Tage bei 26-28 °C gelagert. Jeweils 10 µg Gesamtzell-RNA wurde pro Zeitpunkt in einem denaturierenden Formaldehyd-Agarosegel aufgetrennt und mit den cDNA-Fragmenten der Gene PHI7, PHI20, PHI153 und PHI227 hybridisiert.

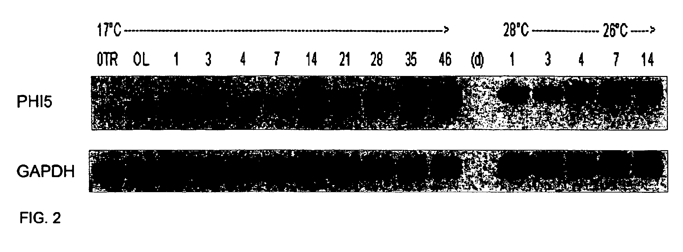

Abbildung 2 zeigt die Induktion der Expression des PHI (postharvest induced) -Gens 5 in Wurzeln nach der Ernte der Zuckerrübe durch ein RT-PCR Experiment. Das PHI5-Transkript ist unmittelbar vor der Ernte (0d) nur sehr schwach in Rübenwurzeln und gering in Blättern nachweisbar. In cDNA-Bibliotheken, welche aus RNA von 1, 4, 7, 14, 21, 28, 35 und 46 Tage bei 17 °C bzw. 1, 3, 4, 7 und 14 Tage bei 26-28 °C gelagerten Wurzeln gewonnen wurden, ist ein starkes PHI5 Transkript nachweisbar. Der Nachweis der Expression des Glycerinaldehyd-3-phosphat-Dehydrogenase-Gens (GAPDH) zeigt, dass gleiche cDNA Mengen für die RT-PCR Reaktionen verwendet wurden.

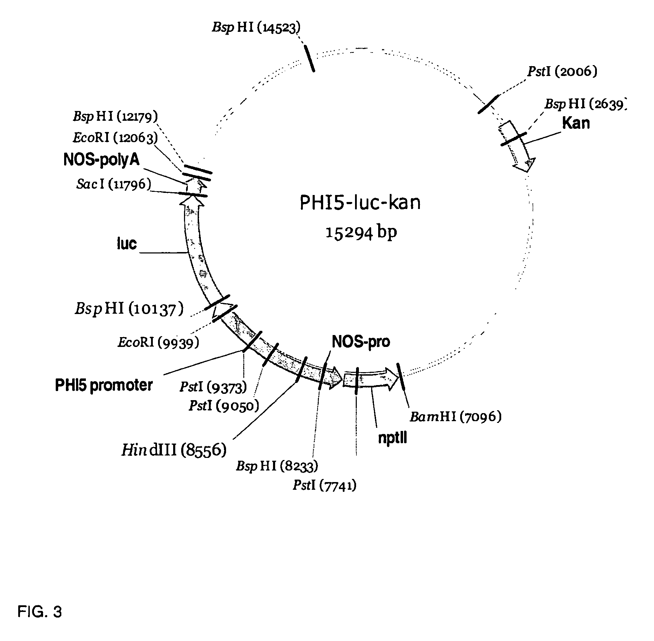

Abbildung 3 zeigt den Reportergenvektor PHI5-luc-kan mit einer translationellen Fusion zwischen dem Promotor PHI5 und dem Luciferase-Gen aus *Photinus pyralis.* Der Promotor PHI5 in dem Vektor PHI5-luc-kan umfasst die Nukleotidpositionen 1-1587 der Nukleotidsequenz SEQ ID NO: 1 und kann mit Hindlll und BspHI aus dem Vektor isoliert und mit anderen Genen kombiniert werden.

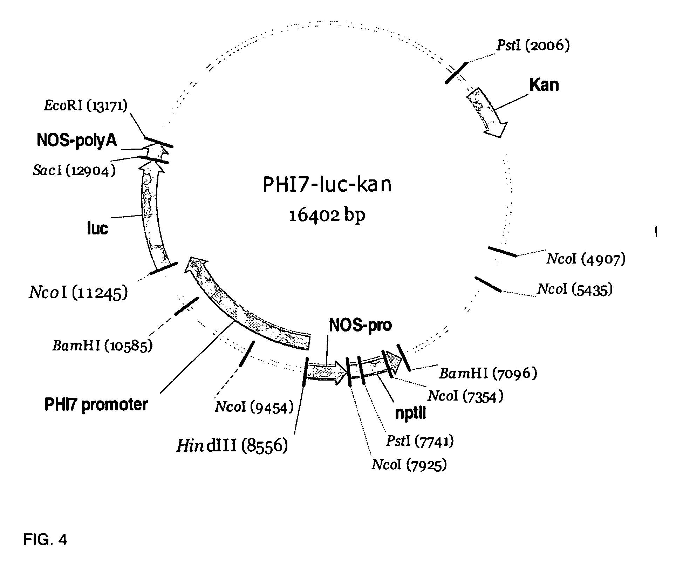

Abbildung 4 zeigt den Reportergenvektor PHI7-luc-kan mit einer translationellen Fusion zwischen dem Promotor PHI7 und dem Luciferase-Gen aus *Photinus pyralis.* Der Promotor PHI7 in dem Vektor PHI7-luc-kan umfasst die Nukleotidpositionen 1-2695 der Nukleotidsequenz SEQ ID NO: 2 und kann mit Hindlll und Ncol aus dem Vektor isoliert und mit anderen Genen kombiniert werden.

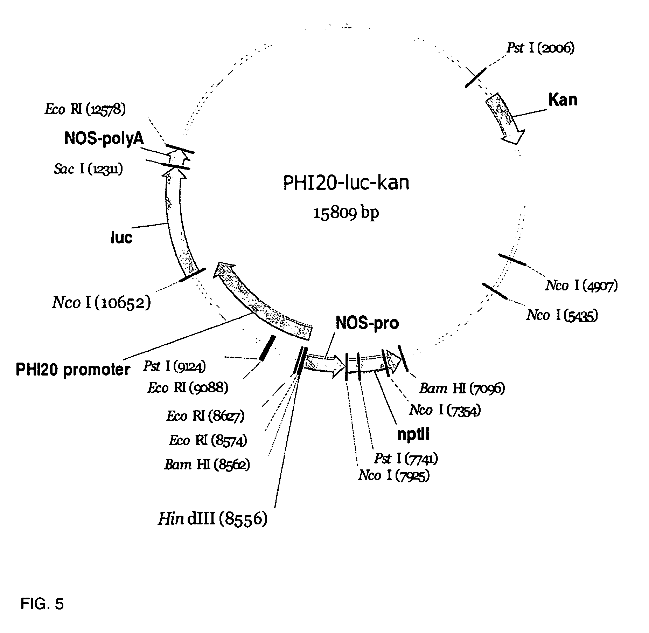

Abbildung 5 zeigt den Reportergenvektor PHI20-luc-kan mit einer translationellen Fusion zwischen dem Promotor PHI20 und dem Luciferase-Gen aus *Photinus pyralis.* Der Promotor PHI20 in dem Vektor PHI20-luc-kan umfasst die Nukleotidpositionen 1-2102 der Nukleotidsequenz SEQ ID NO: 3 und kann mit HindIII und Ncol aus dem Vektor isoliert und mit anderen Genen kombiniert werden.

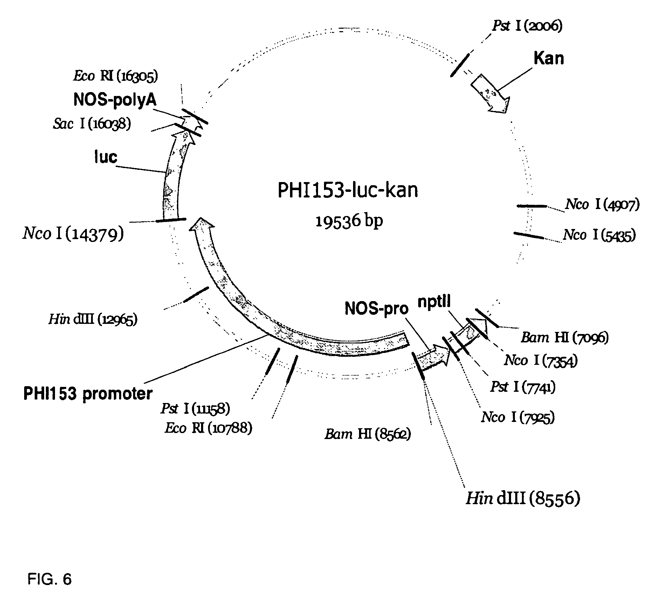

Abbildung 6 zeigt den Reportergenvektor PHI153-luc-kan mit einer translationellen Fusion zwischen dem Promotor PHI153 und dem Luciferase-Gen aus *Photinus pyralis.* Der Promotor PHI153 in dem Vektor PHI153-luc-kan umfasst die Nukleotidpositionen 1-5829 der Nukleotidsequenz SEQ ID NO: 4 und kann mit HindIII und Ncol aus dem Vektor isoliert und mit anderen Genen kombiniert werden.

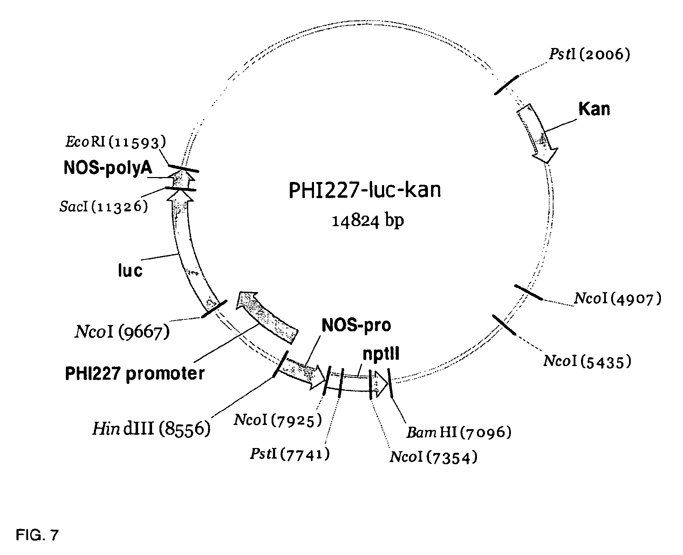

Abbildung 7 zeigt den Reportergenvektor PHI227-luc-kan mit einer translationellen Fusion zwischen dem Promotor PHI227 und dem Luciferase-Gen aus *Photinus pyralis.* Der Promotor PHI227 in dem Vektor PHI227-luc-kan umfaßt die Nukleotidpositionen 1-1117 der Nukleotidsequenz SEQ ID NO: 5 und kann mit HindIII und Ncol aus dem Vektor isoliert und mit anderen Genen kombiniert werden.

### Nachweis der Induktion der Genexpression von PHI 7, 20, 154 und 227 in geernteten Wurzeln der Zuckerrübe

Zuckerrübensaatgut wurde im Feld im Frühjahr ausgedrillt und die Zuckerrüben wurden nach der gängigen landwirtschaftlichen Praxis kultiviert. Die Speicherwurzeln von 24 Wochen alten Pflanzen wurden im Herbst geerntet und durch eine 30 sekündige Behandlung in einem handelsüblichen Betonmischer (Attika) oberflächlich verletzt, um die für eine mechanische Rodung typischen Verletzungen und Quetschungen zu erzeugen. Anschließend wurden die Speicherorgane zum einen bei 17 °C und zum anderen bei 26-28 °C gelagert. Aus den bei 17 °C gelagerten Rüben wurden 1, 3, 4, 7, 14, 21, 28, 35 und 46 Tage nach der Ernte und bei den 26-28 °C gelagerten Rüben 1, 3, 4, 7, und 14 Tage nach der Ernte jeweils 5 Rüben entnommen, und Gesamtzell-RNA wurde nach Logemann et al., (1987) isoliert.

Die Untersuchung der nach der Ernte induzierten Genexpression erfolgte durch eine RNA-Blot-Analyse nach Sambrook et al. (1989). Dazu wurden jeweils 10 µg Gesamtzell-RNA aus Blättern und Rüben, die unmittelbar vor der Ernte (0d) aus dem Feld entnommen worden sind, sowie RNA aus Rüben, die 1-35 Tage lange gelagert worden sind, in einem denaturierenden Formaldehyd-Agarosegel aufgetrennt. Die elektrophoretisch aufgetrennte RNA wurd durch Kapillar-Blot Technik auf eine Hybond N Nylonmembran (Amersham Pharmacia Biotech, Freiburg) übertragen. Die radioaktive Markierung von jeweils 20 ng der cDNA-Klone der postharvest induced (PHI) Gene 7, 20, 153 und 227 mit 50 µCi ³²P-dATP (6000 Ci/mMol, Amersham Pharmacia Biotech, Freiburg) erfolgte mit Hilfe des Prime-It II Random Primer Kit (Stratagene GmbH, Heidelberg) entsprechend der Herstellerangaben. Anschließend wurden die RNA-Filter mit den markierten Sonden hybridisiert, gewaschen und auf einem Röntgenfilm exponiert.

Das Gen PHI7 wurde vor der Ernte in Blättern nicht und in der Speicherwurzel der Zuckerrübe nur sehr gering exprimiert. Nach der Ernte und während der Lagerung kam es zu einer starken Induktion des Gens PHI7 in der Wurzel. Die Induktion war bei 28 °C höher als bei 17 °C (Abb. 1).

Das Gen PHI20 wurde vor der Ernte in Blättern und in der Wurzel der Zuckerrübe nur gering exprimiert. Nach der Ernte und während der Lagerung kam es zu einer starken, lang anhaltenden Induktion des Gens PHI20 in den Speicherorganen. Die Induktion war bei 17 °C und 28 °C vergleichbar (Abb. 1).

Das Gen PHI153 wurde vor der Ernte weder in Blättern noch in der Speicherwurzel der Zuckerrübe exprimiert. Nach der Ernte und während der Lagerung kam es zu einer starken Induktion des Gens PHI153 in der Wurzel. Die Induktion war bei 28 °C vergleichbar mit der bei 17 °C (Abb. 1).

Das Gen PHI227 wurde vor der Ernte in Blättern und in der Wurzel der Zuckerrübe nur gering exprimiert. Nach der Ernte und während der Lagerung kam es zu einer starken Induktion und einer hohen, lang anhaltenden Expression des PHI227 Gens in der Wurzel. Die Induktion war bei 17 °C und 28 °C vergleichbar (Abb. 1).

### Nachweis der Induktion der Genexpression von PHI5 in geernteten Wurzeln der Zuckerrübe

Für den Nachweis der Induktion der Expression von PHI5 nach der Ernte der Zuckerrübenwurzel wurde ein RT-PCR Experiment durchgeführt. Aus jeweils 5 µg Gesamtzell-RNA von Blättern und Wurzeln, die unmittelbar vor der Ernte (0d) dem Feld entnommen worden sind, wurde jeweils eine cDNA-Bibliothek mit Hilfe des RevertAid H Minus cDNA Synthese Kit (MBI Fermentas) entsprechend der Herstellerangaben hergestellt. Weitere cDNA-Bibliotheken wurden jeweils aus RNA hergestellt, die aus 1, 3, 4, 7, 14, 21, 28, 35 und 46 Tage bei 17 °C und 1, 3, 4, 7, und 14 Tage bei 26-28 °C gelagerten Rüben isoliert worden sind. Die Expression des Gens PHI5 Gens wurde mit den Primern PHI5-1 (GTG CAA GGA TTC TGG CAC CCG TCG GTG G) (SEQ ID NO: 6) und PHI5-2 (GTA TGG GCC GCG GCA GAT CCA GGT AGC G) (SEQ ID NO: 7) mit Hilfe von Taq-Polymerase (Q-Biogene) entsprechend den Herstellerangaben nachgewiesen. Zu Kontrollzwecken wurde die Expression des Gens Glycerinaldehyd-3-phosphat-Dehydrogenase (GAPDH) durch die Primer GAPDH-1 (ATG TTT AAG TAC GAC AGT GTT CAC G) (SEQ ID NO: 8) und GAPDH-2 (ATG TGA AGG TCT GAC TTG TAT TCG T) (SEQ ID NO: 9) detektiert, um sicherzustellen, dass gleiche cDNA-Mengen für die RT-PCR eingesetzt worden sind.

Die RT-PCR-Untersuchung zeigt, dass vor der Ernte (0d) das Gen PHI5 in Blättern schwach und in Wurzeln sehr gering exprimiert wird. Die bei 17 °C und 28 °C gelagerten Rüben zeigten eine starke Expression und damit eine Induktion des Gens PHI5 in dem Speicherorgan nach der Ernte und während der Lagerung (Abb.2). Die bei 17 °C gelagerten Rüben zeigten 1-7 Tage nach der Ernte eine deutlich geringere Expression des PH15-Gens als während der Tage 14-46. Die bei 26-28°C gelagerten Rüben zeigten am Tag 1 und 3 nach der Ernte eine deutlich geringere Expression des PHI5-Gens als während der Tage 4, 7 und 14. Die Expression des GAPDH Gens war in allen Proben gleich (Abb.2). Diese Ergebnisse zeigten, dass die Expression des PH15-Gens nach der Ernte induziert wird. Allerdings war die Induktion während der ersten Tage der Lagerung, die physiologisch durch die Folgen der mechanischen Rodung bestimmt wird, deutlich geringer als während des Phase der späteren Lagerung und der damit verbundenen physiologischen Veränderungen.

### Klassifizierung der PHI Promotoren

Die Promotoren PHI5, PHI7, PHI20, PHI153 und PHI227 wurden alle nach der Ernte in der Wurzel der Zuckerrübe durch den Erntevorang und die anschließende Lagerung des Speicherorgans induziert (Abb. 1 und 2). Neben dieser generellen Eigenschaft der nacherntlichen Induktion unterscheiden sich die fünf Promotoren im Hinblick auf die Grundaktivität vor der Ernte, dem Einfluss der Rodung und der Lagerung auf die Kinetik der Induktion und den Einfluss der Lagerungstemperatur auf die Stärke der Promotorinduktion. Diese Unterschiede erlauben es, vier Unterklassen von nacherntlich induzierten Promotoren zu bilden (Tabelle 1).

Der Promotor PHI153 gehört zu der ersten Unterklasse von PHI-Promotoren, bei denen vor der Ernte kein Gentranskript in dem Speicherorgan nachweisbar war.

Die Promotoren PHI20 und PHI227 gehören zu der zweiten Klasse von PHI-Promotoren, die eine schwache Aktivität vor der Ernte in dem Speicherorgan zeigten, deren Induktion nach der Ernte sowohl bei niedrigeren (17 °C) als auch bei höheren Temperaturen (28 °C) gleich stark und damit temperaturunabhängig war.

Der Promotor PHI7 gehört zu der dritten Unterklasse, deren Promotoren vor der Ernte eine schwache Aktivität in dem Speicherorgan zeigten, deren Induktion bei höheren Temperaturen (28 °C) jedoch deutlich stärker war als bei niedrigeren Temperaturen (17°C).

Der Promotor PHI5 gehört zu der vierten Unterklasse von PHI-Promotoren, die eine schwache Aktivität vor der Ernte in dem Speicherorgan zeigten, deren Aktivität infolge der mechanischen Folgen der Ernte moderat und während der Lagerung stark anstieg.

### Fusion der Promotoren PHI5, PHI7, PHI20, PHI153 und PHI227 mit dem Luciferasegen aus Photinus pyralis

Um die Aktivität der Promotoren PHI5, PHI7, PHI20, PHI153 und PHI227 in Zuckerrüben nachzuweisen, wurden die Promotoren mit dem Luciferasegen aus *Photinus pyralis* translationell fusioniert und in Zuckerrüben transformiert. Dazu wurde der Promotor PHI5 als HindIII-BspHI Fragment und die Promotoren PHI7, PH120, PHI153 und PHI227 als Hindlll-Ncol Fragment in den mit HindIII-BspHI bzw. Hindlll-Ncol linearisierten binären Vektor pGPTV-kan (Becker et al., 1992) kloniert.

Die entstandenen Vektoren tragen die Bezeichnung PHI5-luc-kan (Abb. 3), PHI7-luc-kan (Abb. 4), PHI20-luc-kan (Abb. 5), PHI153-luc-kan (Abb. 6) und PHI227-luc-kan (Abb. 7). Die binären Vektoren wurden in den *Agrobacterium tumefaciens* Stamm C58C1 mit dem residenten Plasmid pGV2260 durch ein direktes DNA-Transformationsverfahren (An, 1987) transformiert. Die Selektion rekombinanter *A. tumefaciens*-Klone erfolgte unter Verwendung des Antibiotikums Kanamycin (50 mg/l).

Die Transformation der Zuckerrüben erfolgte nach Lindsey et al. (1991) unter Verwendung des Antibiotikums Kanamycin. Die Transgenität der Pflanzen wurde durch PCR überprüft. Die Verwendung der Primer GTGGAGAGGCTATTCGGTA (SEQ ID NO: 10) und CCACCATGATATTCGGCAAG (SEQ ID NO: 11) führte zu der Amplifikation eines 553 bp großen DNA-Fragments aus dem nptll-Gen. Die PCR wurde unter Verwendung von 10 ng genomischer DNA, einer Primerkonzentration von 0,2 µM bei einer Annealingtemperatur von 55 °C in einem Multicycler PTC-200 (MJ Research, Watertown, USA) durchgeführt.

Das Luciferasegen des Vektors PHI5-luc-kan (Abb. 3) kann als BspHI-Sacl-Fragment und das Luciferasegen der Vektoren PHI7-luc-kan (Abb. 4), PHI20-luc-kan (Abb. 5), PHI153-luc-kan (Abb. 6) und PHI227-luc-kan (Abb. 7) als Ncol-Sacl-Fragment freigesetzt und durch ein anderes zu exprimierendes Gen wie z.B. einem Invertaseinhibitorgen ersetzt werden. Dazu sollte das zu exprimierende Gen ebenfalls als Ncol-Sacl-Fragment vorliegen. Alternativ kann der PHI5-Promotor als Hindlll-BspHI-Fragment und die Promotoren PHI7, PH120, PHI153 und PHI227 als Hindlll-Ncol-Fragment isoliert und in geeignete Expressionsvektoren eingefügt werden.

### Nachweis der PHI5-Promotoraktivität in gelagerten Wurzeln der Zuckerrübe

Transgene Zuckerrüben, die mit dem Reportergenkonstrukt PHI5-luc-kan transformiert worden waren, wurden unter Gewächshausbedingungen angezogen. Zwanzig Wochen alte Pflanzen wurden geerntet und die Wurzeln für sechs Wochen gelagert. Die Aktivität des Promotors wurde in Wurzeln und Blättern vor der Ernte und wöchentlich in den gelagerten Wurzeln durch eine Reportergenmessung analysiert.

Die *Photinus pyralis*-Luciferaseaktivität wurde mit dem Luciferase Assay System (Promega, Mannheim, Deutschland) in einem Sirius Luminometer (Berthold Detection System GmbH, Pforzheim, Deutschland) entsprechend den Herstellerangaben bestimmt. Für die Gewinnung eines für die Messung geeigneten Enyzmextraktes wurde zunächst das Gewicht der Gewebeproben ermittelt. Die Blattproben wurden unter Zugabe von Seesand mit dem 10-fachen Volumen (v/w) an Passive Lysis Buffer (PLB) in einem Mörser und die Wurzelproben in einem handelsüblichen Küchengerät (Warring Blender) homogenisiert. Der flüssige Überstand wurde in ein 1.5-ml-Eppendorfgefäß überführt und 5 min bei 4 °C und 20 000 g abzentrifugiert. Der klare Überstand wurde abgenommen und jeweils 10 µl Rohextrakt für die *Photinus*-Luciferaseaktivitätsmessung eingesetzt.

Während der PHI5-Promotor vor der Ernte in Wurzeln und Blättern nur schwach aktiv war, nahm die Promotoraktivität in den geernteten und gelagerten Wurzeln von 8 unabhängigen Transformanten stark bzw. sehr stark zu. Die PHI5-Promotoraktivität wird gemäß den Ergebnissen der Reportergenstudie in Übereinstimmung mit den RT-PCR Ergebnissen nach der Ernte in der Wurzel der Zuckerrübe stark induziert.

### Transgene Pflanzen mit besonderen Eigenschaften

Mit dem erfindungsgemäßen Promotor lassen sich transgene Pflanzen mit besonderen Eigenschaften herstellen:
a) Verbesserter Kohlenhydratmetabolismus in Speicherorganen nach der Ernte
b) Verbesserter Stickstoffmetabolismus in Speicherorganen nach der Ernte
c) Verbesserte Trockenstressresistenz und verbesserter Wasserstatus in Speicherorganen nach der Ernte
d) Verbesserte Kälte- und Frosttoleranz von Speicherorganen nach der Ernte
e) Erhöhte Resistenz/Toleranz von Speicherorganen gegenüber Pathogenen nach der Ernte
f) Verbesserter Sekundärstoffwechsel in Speicherorganen nach der Ernte

### Verbesserter Kohlenhydratmetabolismus in Speicherorganen nach der Ernte

Der Kohlenhydratmetabolismus der Speicherorgane von Pflanzen kann durch die Verwendung der erfindungsgemäßen Promotoren in vielfältiger Hinsicht verbessert werden. Durch die mechanische Behandlung während des Rodens und die physiologischen Veränderungen während der Lagerung von Zuckerrüben und Karotten kommt es nacherntlich zu einer verstärkten Hydrolyse und Veratmung von Sucrose und der Akkumulation von Invertzucker (Burba 1973, Smed et al., 1996, Galindo et al., 2004). Die entstehenden Zuckerverluste in der Größenordnung von 0.01-0,025 % pro Tag reduzieren die isolierbare Zuckermenge aus Zuckerrüben. Die Invertzuckerbildung führt zu technologischen Schwierigkeiten während der industriellen Zuckerisolierung (Burba, 1976).

Die erfindungsgemäßen Promotoren können genutzt werden, um die Sucrosehydrolyse und Invertzuckerbildung zu reduzieren. Dazu werden geeignete Invertaseinhibitorgene nacherntlich verstärkt exprimiert oder die Expression des oder der endogenen Sucrosesynthasegene oder Invertasegene werden durch einen Antisense- oder RNAᵢ-Ansatz reduziert.

Die erfindungsgemäßen Promotoren können auch genutzt werden, um höhere Ausbeuten an Polyfruktanen wie Inulin und die Produktion von längerkettigeren Inulinmolekülen in der Zichorienwurzel zu erreichen. Dazu wird der Abbau von Inulin in der geernteten Zichorie reduziert bzw. verhindert, indem die Aktivität der Fructosyl-Exohydrolase in der Wurzel nach der Ernte durch einen Antisense- oder RNAᵢ-Ansatz reduziert wird.

### Verbesserter Stickstoffmetabolismus in Speicherorganen nach der Ernte

Der erfindungsgemäße Promotor kann genutzt werden, um den Gehalt von extrahierbarem "schädlichen Stickstoff', wie Aminosäuren, in den Speicherorganen der Pflanze nach der Ernte zu reduzieren. Erhöhte Konzentrationen an N-Verbindungen in Speicherorganen reduzieren oft den ernährungsphysiologischen Wert von Ernteprodukten oder erschweren die Isolierung von Speicherstoffen wie Sucrose aus Zuckerrübenwurzeln. Durch verstärkte Expression entsprechender Enzyme, Transkriptionsfaktoren, Speicherproteine und dergleichen lässt sich in den Speicherorganen ein verstärkter Einbau von Aminosäuren in Proteine erreichen. Während der Zuckergewinnung können Proteine dann im Gegensatz zu nicht extrahierbaren Aminosäuren aus der Zuckerrübe gefällt werden.

### Erhöhung der Toleranz von Speicherorganen gegenüber bodenbürtigen Phytopathogenen Pilzen und Bakterien

Der erfindungsgemäße Promotor kann auch genutzt werden, um in Kombination mit einem Gen oder einer Genkombination eine direkte oder indirekte, antifungale Wirkung in den Speicherorganen von Pflanzen auszuprägen. Die antifungale Wirkung führt zu einer erhöhten Pilzresistenz oder Pilztoleranz nach der Ernte und während der Lagerung.

Der Promotor wird hierzu mit Genen der Pathogenabwehr, deren Genprodukte eine direkte oder indirekte antifungale bzw. antibakterielle Wirkung haben, translationell oder transkriptionell fusioniert. Die Promotor-Gen-Kombinationen werden in den binären Transformationsvektor pGPTV kloniert und durch *A. tumefaciens* vermittelte Transformation in Zuckerrübe, Karotte oder Kartoffel transformiert. Die Transgenität der Pflanzen wird wie beschrieben durch PCR überprüft und die Expression der Gene in den Wurzeln bzw. Knollen durch RNA-Blot-Studien verifiziert. Die erhöhte Pilzresistenz bzw. Bakterienresistenz der Speicherorgane wird bei Resistenzprüfungen beobachtet.

Überraschenderweise führte die nacherntlich induzierte Expression von Pathogenabwehrgenen nicht zu der bei einer konstitutiven Expression während der vegetativen Entwicklung der Pflanze oft beobachteten Kleinwüchsigkeit oder Ertragsminderung (Heil and Baldwin, 2002).
SEQ ID NO: 4: Translationstart-ATG: 5826-5828

### Referenz:

Altschul, S.F. et al. (1990). Basic Local Alignment search tool, J.Mol. Biol. 215: 403-410
An, G. (1987). Binary Ti vectors for plant transformation and promoter analysis. Methods Enzymol. 153, 292-305.
Becker D, Kemper E, Schell J, and Masterson R. (1992). New plant binary vectors with selectable markers located proximal to the left T-DNA border. Plant Mol Biol. 20(6):1195-7.
Burba, M. (1976). Atmung und Saccharosestoffwechsel lagernder Zuckerrüben. Zeitschrift für die Zuckerindustrie 26: 647-658.
Galindo, F. G., Herppich, W., Gekas, V., and Sjöholm, I. (2004). Factors affecting quality and postharvest properties of vegetables: Integration of water relations and metabolism. Critical Reviews in Food Science and Nutrition 44:139-154.
Heil, M. and Baldwin, I. T. (2002). Fitness costs of induced resistance: emerging experimental support for a slippery concept. Trends Plant Sci. 2002 Feb;7(2):61-7.
Lindsey, K,. Gallois, P., and Eady, C. (1991) Regeneration and transformation of sugar beet by Agrobacterium tumefaciens. Plant Tissue Culture Manual B7: 1-13; Kluwer Academic Publishers.
Logemann, E., Parniske. M., Hahlbrock, K. (1995). Modes of expression and common structural features of the complete phenylalanine ammonia-lyase gene family in parsley. Proc Natl Acad Sci USA. 1995 92(13):5905-9.
Nilsson, T. (2000). Postharvest storage and handling of vegetables., In Fruit and Vegetable Quality (an integrated view), Seite 96-121. Herausgeber Shewfew, R. L. and Brückner, B., Technomic Publishing Inc.
Sambrook, J., Fritsch, E..F., and Maniatis, T (1989). In Molecular Cloning, A Laboratrory Manual (Cold Spring Harbor Laboratory Press, New York).
Smed, E., Augustinussen, E., and Stensen, J. K. (1996). Loss of sugar in injured sugar beet losses from lifting , storing and washing. Proceedings of the 59th IIRB Congress, 533-545.

### SEQUENZPROTOKOLL

### Organization Applicant

Street :
City :
State :
Country :
Postalcode :
PhoneNumber :
FaxNumber :
EmailAddress :

<110> organizationName : KWS SAAT AG

### Organization Applicant

Street :
City :
state :
Country :
Postalcode :
PhoneNumber :
FaxNumber :
EmailAddress :

<110> organizationName : südzucker AG Mannheim/ochsenfurt

### Application Project

<120> Title : Lagerungsinduzierter Promotor
<130> AppFileReference : KWS 0091 PCT/EP/D1
<140> CurrentAppNumber :
   <141> CurrentFilingDate : -------⁻---⁻---

### Earlier Applications

<150> PriorAppNumber : DE102004057291.7
   <151> PriorFilingDate : 2004-11-26

### Earlier Applications

<150> priorAppNumber : EP05817889.8
   <151> PriorFilingDate : 2005-11-23

### Earlier Applications

<150> priorAppNumber : PCT/DE2005/002093
   <151> PriorFilingDate : 2005-11-23

### Sequence

<213> OrganismName : Beta vulgaris
<400> PreSequencestring : 
aagcttttct ttattgaata tacatataac accgtgcaca tacagatagc aacgatttga 60
aggctggtgg tagtaaagta tctataagta aaaggaaagc aatccaagta ctttgttttg 120
aaagccctcg atctagccta tagaaaaggt agttgactta cttagttaaa gcaaagcatt 180
aagaaaggaa tttgattgat atggatactt ttttgaaaag tgagacactt tctatgccga 240
gtgtaaattc gatacctcct tgcttccctt aaagctcaac ctccccatgg tatgccctcc 300
tctggattgg gtagtccaac accctgaagt taaagaataa tggttaaaca tttctgattt 360
taaaggggat tacctataaa ttcaataagt ggtctaatac atgaccgtta ttgtctttta 420
agttctggta acattaagaa tttctttatt tactttggta agttcgagga tagtttaagc 480
cttaaaaagg ctgcagacct gtgcgaggta atgaacaagc tccagagatt tttcatttaa 540
ttttcatggc tgatttgtca tttgtatatt taatttcaga tttgtaattt ttgtatgtag 600
attatatttt ttttagtttg gaattaatag ggatgtattt cactgcattt ttagttgtat 660
gccagtgggt atttttgatt ttagtttggg atgtatgtgg ctgcaaacat gtgtgtattt 720
tggtggaaag tggtggaaat gtggggggga ggggtagtgt agacctgcaa atgtgattgt 780
 
tgcttttgt tttggtagtg cagacctata ggcctgcagg tctgtaactt tttttcgac 840
atacatttca acaaactgat gtgatttttc ttaagaaccg catcaataaa tcatttactg 900
attcgatttt tgatcggatc agtaaatgat tgggagagct gctgcgagcc cacctgatgc 960
ggaccaaccc aattttgacc acatcaagat gggctttttt ccactaatgt aagatcatat 1020
attatctaga agtgagcccc ttaacttgta aaatgaccct tttcacttac aaagtaatta 1080
acccttaaaa aaaataaagt gaggtggtct taccataatt ttattgtaag acttccttgc 1140
tcaagatcta ctaataatga gaatatgcca agaaaaaaac gactatgaga cgattccata 1200
atcctcgaaa gttcttaaaa tcttaaacta aaacatttag gaaaaaaatt ctgaaaaaat 1260
ttcaacgtaa cctacaaagc tccttcaaac aactatgttt taatcagcac gaaaccttta 1320
ttaaacctca ttcagtcatc cccttctaac aagatagtcc tcttacagaa taaaaataca 1380
caagaattca tcctcatccc tcgtcagcaa tatgccaaat ctatctttga caattctaca 1440
caatcaaaca atgaatattg cccgaaaaca aagcaaagaa gatcaagtta gccgcctaca 1500
accccctattt aaaccccccct catcatctca acatttccac acacaaaaac catactacta 1560
aatcatcatc ataatataca atcatga 1587
<212> Typs : DNA
   <211> Length : 1587
   SequenceName : 1
   SequenceDescription :

### Feature

Sequence: 1:
<221> FeatureKey : Translationstart-ATG
<222> LocationFrom : 1584
<222> LocationTo : 1586
   Other Information :
   CDSJoin : No

### Sequence

<213> organismName : Beta vulgaris
<400> Presequencestring : 
aagcttctcg agaaagccat ctaaaaggag taggagagag agaggaaggc attgacgagt 60
tgagaactga gttggatcgt tcggatttct tcttcttcct gagcgtactt ttaacacccg 120
ccattactcg ttagcaatga agaagagaag tagcacgagt ttcttacatc ttgaaagaga 180
gtcaatggtt ttggttggtg cgttacgaac gagatgggat gggactatgg tctatgggag 240
aatggtcaac tagacccaaa tgctaatgcg atggattagg ttatttaggt cccgttcttt 300
ttcactaaga taaaatgaac tgaaataaat tgaactaata gaaactatat tatagagaga 360
tattaaacta aattgaactg aaattaactg gctgaaaata aatccaaaat aacagagcct 420
taaccttagt ggtgaagtgg cggaatgaag atttttgtat taaggtctaa ctcagacagt 480
tagagcttcc gtcacgtaat caagagactg gaccaatgat aatcaaacgt ctcgcgccat 540
tgctcttgaa caagatttca cacatgtgaa gatagcggat tttcgaaaca catcggcata 600
ttgccaaggc cttaaaaccc tctctgatta gttaaatagt gtgagggacg gtgtggcggt 660
gtctggtagt cgccttctcc tacacatggt ggttgggttt accgaggcct acaagggtgt 720
tggcaccatg attcggcaaa gtaaaccttt gccttccttc attaaagcac gttcgagcct 780
tctccctaaa ggaaatgtca atgcttgatg aatcaccgtt ggctatggtg gtgactcaga 840
acgaggatgg cgcatctgac tccttttctc atactcgaca tggtaagaaa gtatggaacc 900
atggctcgca caataatcat aagcaatctg gtggtggtcg cagcgatgga aagtgtcgtg 960
gtggtggtgg tcgtgtccgc ggtggtcacg gtggaggcgc cggacaacaa caaccttagg 1020
ccgccacccc tccttggtcg tatgcagctg gtagttggtg ttgggtgccc caacagtggg 1080
tagttccttt ttacccacac tcaacagtgg gcagttccgt tattgggcca tatccaaata 1140
gtctagtaag tcaacgaggt ttgagtatgc cgggcggctg ggcctgcgtg catctcaagt 1200
ctatatggtt gcatatgttc ctactgatct tacttttgca tttcacacta tgacacttgc 1260
ttctccgaac tccaattggt atatggacat tggtgctacc tcacatatga cctccacacc 1320
aagtaatctc acgtcttatt ttaatttgag caatacaaat ggtattacta tttgtaatgg 1380
tctaacaatt ccgatttgtg gttatggtaa aaaaattccg atttttgtgg ttatggtcat 1440
tcacatatat cttctttcac aaaccaaaca gaggaaagtg aagaaaataa ttttactcaa 1500
aaatattttc cttccaatcc gtcaaaaaga atctcgtgat tccttaacaa aaaaaaaaaa 1560
attaaaaaaa aaacaaaggg aaaaatagtg tattgtaggg catacgtaga ataatacggt 1620
tgactagaga tggcaatgga tcatgcaacc gacccattta tatgggtctg agtctagata 1680
ttttagaccc aatgggtccg ggtcgggtct gggtccacat atgttaggca ccggcttgat 1740
tcgcagacct atttacatat tagaattttt tttacctata ctctaaagtt ttattagttg 1800
tgattttatt aactcgttag tgttgtaact ccatataaga cttgtaactt tgtcaattgt 1860
aacattttat gatttcatga gttaattttt aaaatttgt ttagataata cttaaaccag 1920
ggatgcaaga cctatatagt agggcagaat tttttaatta catatattca aaaatgtaat 1980
gaaaacttag aattatgatg gacacattta ggacccaaat aggacccgtg gatccgctag 2040
attcaccgaa tctggaactg ggtctgaaaa attttgaccc aacgaattta aaatggatat 2100
gaatctgtaa aaaaataaac gggcatgggt tcagttgaac tcgccgcaga ccctccccat 2160
 
tctacgttga cggtcacgga tggtgcatga aaggggtcaa cgatcaatgt gagagcaacc 2220
caagcgtttg gtttcccaat ttccactatt ttcgcaaatc atttcactgt aaacatttaa 2280
gaaaaaacgg tgaacattcc agactcaact agacaactag ttggcttgaa ccttctcgaa 2340
ttattttctt aacaaaaaga aagttcatcc tcaaacgcac agtttcatat ccataacgcc 2400
acaacacaca aaaaacgcgt cgtttcctca caacaatttt taaaaaaccc caccaaaaat 2460
aatactagta taacacaact aagaaacaat tctagagaag agtagaactc tccagaacaa 2520
agaagcaaga aaatagctct cctctctgct ataaaaacct cttcctgtct ttctcgcctt 2580
catcaccatt ctctctctaa agcaatctaa gcaaacacca aagcaatcaa tccacattat 2640
ctcttctata atcgcgaaat ttctaggtta tttttttctg aaggtgcatc catgg 2695
<212> Type : DNA <211> Length : 2695
   SequenceName : 2
   SequenceDescription :

### Feature

Sequence: 2:
   <221> FeatureKey : Translationsstart-ATG
   <222> LocationFrom : 2692
   <222> LocationTo : 2694
      Other Information :
      CDSJoin : No

### Sequence

<213> organismName : Beta vulgaris
<400> PreSequenceString : 
aagcttggat ccatcgatga attcggcgcg ccactagtat attggggttg aggagacaac 60
cattaacaaa cgaattcaaa attttaattc ttcttatatt tatatatgta tttctgtttc 120
atcttcattt ctttttttgc acaatcctat taaaatctca tattcaatga aattcggcta 180
attcaatcaa gagatattca acactatgtt caattcctcc tcaatgtatg cacccaactc 240
caagcgatcc aactaataag gtctgttcgt aatcattttt tgttttcaat tttctgtttt 300
tctgaaaact aaaaacaaaa aacagaatac tagaaaaagt gattttcaat cacattttag 360
ttatcagttt tcagaatcat catgttttca ataagttcca ttatttttg gttcattata 420
ggacatatag taatattata tgacttctaa aaacaaaaaa ctgaaaacca cagtgataac 480
gaccgggccc taagttacga gtttacaata ggcaacaacg caacattaat tagaattcac 540
ccggttatat catatacccg cgtgctgcag gtggcttaca cttatatatg atatagccgt 600
tcgagggcac cttaaggctc accctccttt cgaagacttc atgcatgcgc attcaggaat 660
ccatcgcacc atcaaggctc atcgaacacc aggggcagat gtacattgta ctgtgcttag 720
tgcggttagt tgacctcact taaatttgaa aacttcggga actttcttta cataatttga 780
gatttttttg gatatttcct agtaaaattg attgtttgaa cctactaaaa aatagtaatt 840
ttatttcgac ctcactgaat acatattcta gatccatcaa tgaacaaaaa cgccacgcat 900
cgagtcagag gaaggatctg ctgctatagt gttattgtct cttgctcgct agaacattgt 960
ccacataaaa agcatcgtcg acgtccatca catgcatgct gctcatggag cctacctaat 1020
tgttgcggat gctactcagc cctccatagg aagtggctta gttgaagggg gacctaggtc 1080
atgaactacg ttattatcag ttcacgtgct agtacttgag ataatgctac atggacataa 1140
gtccataaca catgataaat caatcgtacg tgaaagggtg gcggctacct ttttctaatt 1200
gtatttgtgt cactactttt tctcttctgc tcatcatcat atcgtaagtc aaaaattcgt 1260
gatgactaat gtacgtctag tcatgcaagt ttattaatta ggaaaaatta tttttgataa 1320
gataactttt gcgcgttttt cttaaattaa gttaattttt tgattaaata tgaataaatt 1380
aattttatat ctacttttgt tataaatgag ttaaatcaca atgtcatcct attaaaatgt 1440
gacacgtatc atactttcaa tgttatgaag tagatacaaa gttaacttat taatcagaag 1500
attaccttat taaaaacaat ttttcttatt aactgtgaac gttattatta atacataata 1560
gtaaacatta ttatcatgtg aaatcgtatt aaatttattt caattagatt taagttaata 1620
actttaattt atataatttt attaataaaa attaaagata tatattaaca gtcaaataaa 1680
cgtattaaaa accattaaaa aaaagataaa ctaatagaat ctccaaggtg gtaaagtcta 1740
accaacgacg aataaacaat tccattatac tagcacgata gataaggtta agttaccata 1800
cttattcatg tgatgtgagt gacatgtgac taaagttacc atgttggcac accatgctac 1860
gtagatttag aaaagtcata cacccgacaa tcaactttaa tttggttgca tgattaaaac 1920
gacgccatta gaaaaaaaat ctaagcaaca tatagtcata tacctccaaa ctttgcattg 1980
attgggttca ctataaataa agaaaagcct ctcactcata aatttcatca aatcttgctt 2040
gaaaataaat ccattaacct aattgagatt cttatagcaa gttttgcata tatagaccat 2100
gg 2102
<212> Type : DNA <211> Length : 2102
   SequenceName : 3
   SequenceDescription :

### Feature

Sequence: 3:
   <221> FeatureKey : Translationstart-ATG
   <222> LocationFrom : 2099
   <222> LocationTo : 2101
      other Information :
      CDSJoin : No

### Sequence

<213> OrganismName : Beta vulgaris
<400> PresSquenceString : 
aagcttggat ccacatgatg atgcttctgg taatttatag tccaactagt cagtttattt 60
atttagaatc tttgtcctat ctttccgtca tttacccctc tttttttttt ttcttttctt 120
ttagggtctc tttcgaaaat ttcatgccta tgctcggctc tcatacaacg tcactcatct 180
agaggcccaa caagtcccaa cctcccctc caatattgaa atttgctttc accaagattt 240
gaacttcgat ctccaatgaa agagataaaa aatcatatca ttgaacttaa ggattcttgg 300
tgtccatcat ttgcatctta gcttcaatgc tttgattatt atactattta cggtttttc 360
accagatccc tccaacaaat caataaattc accatatacc ctcaatgttt cagaaatgca 420
ccgtataccc ttgagtttcg aaaactatgc accagatccc cttttcctaa ctggcattaa 480
cccaccgtta gattatttac gattttacca tcattaagcc ctaatcctaa tttaacctaa 540
ccctaatcct accccctaaac ccttcccctt accctaaccc taccccttgg cagcccccac 600
accacccctc ccctccaccc caccccagcc cctaccctcc ccctccccct cccccaccgc 660
tgatgtccgt gccagcctcc cccaccccca ttgtcgctgc ttttgccggt ctcccccacc 720
ccatcgacgt tgcgcatgct ggcctcccca cctcctgcgc gtctgttcac cgctgacgac 780
tcctcacccc ttcccttcga acaccaaacc catacccctc cccttcgaac accggctaac 840
atcggctacc ttcgaatacc ccacccatcc cccttcgaac accggctaat ttcgaacacc 900
aaacctcccc cacaccaccg gtctccccca cccctcccac acccttgaat ttcatcagtt 960
taaacttcta catattctag attttttaaa aattccttca agtttagaaa aacaataata 1020
gaacagagtt aattagtaaa attaagaaat ttttggttcc ttaatataaa atgtacaacc 1080
acgaagagac catccctatt ccgcatcaat atggaagagc catgtagccg ttaaaaatat 1140
ttgctttatt tgttgtttag agagcaaagt gtattatatt taaccctaaa ctttacggcc 1200
actaatggat attccctcta cgccgtatta ttttatacgt aattacgtat gcactgttat 1260
ataacctacc ttgaccatat ttgtctcgta acatataaag atatatgtta ggtataagat 1320
accaaggatc tttggtccag tagtatggct tcctatcttc gacatgggag accaaagttc 1380
gaatcttggt tgaagcaaaa tttcaatatt gttggagagg aggggggggg ggttgggctt 1440
gttgggcctc tgggtgagtg agtggccctg tgtgagggca gcccaaagaa aattcatctt 1500
accacgggtt ctcgaaagga gtccaaaaag acaatatata tattaggtat aagatattgt 1560
tggatttgtg gtaatgtata catatcaaaa tatcaacatt ctataacttt taataatcca 1620
ctagtattgc tgcggtgatt ttgttgcgat tttacccata aacagcaata tatcacgtaa 1680
aaagaaaagg aaaaaatgta aaataagcag caatatggtt tcaagaaaat tttttttgaa 1740
aaattttaac tgtttgacaa aacatattga tgcggtttct aagaaagaac cgcatcaata 1800
tattaaaata atatcaaaca atattctttg aacatattgt tgcaattcgt acaaaatatc 1860
gcagcaataa taggctttta aagggtccat ctagggtttt cttgttaagt tagtggatgg 1920
gtgtcgttta attctctcat ttactcggct agggtttcac tcctctcact ctctctcctc 1980
ctctcatcag ttttacgcct catcttctct ctttctctct ttgtatcatc agtttcacgc 2040
ctcatcttct cttaatcacg acgcaatgct ttaaccacca tgtactgcta actcgaaacc 2100
aggtcatcgc ttcaattgac tacaccgttt cttctccctc tcttttgtcc tcaccatgac 2160
gaagcaacac gctccatctt cgttcaacga gaaacgcgct aacatcccat agcgaagaaa 2220
atacctgaag atgaattcgg tctccgatga gtttggcggg ttatctttgt ttatcaattt 2280
tttttttttg tttttagat ctgtttctta attttttcg ttatcatagc ggtggtctgt 2340
agtcatgatg tttgtatatc tcttttcttt tctttttttt gggggtgaa ggcagtctgt 2400
ttttttttag gttagtgtta taggtagatc tattagtcaa attttgttat aaattctgta 2460
agaatatggt tctataaaat atatgagtgt aatgatgtaa gtttgtgttt tagttaagtg 2520
ttttatttga cactatctct tatcgtatat gaatatatat caaaaaacaa atttcttttt 2580
gagttgtagt acatatagct gcagtttttg gtaaaaaccg cagcaataag ctctaaatat 2640
ttttaataaa aaataaacct attgctgcgg ttttggatag gaaccgcagc aatagctcgg 2700
cttattgctg tggtcagaaa ccatgacaat atgctaatta cctattgttg catccttaat 2760
tgctacgcgg ccaaaattgc agcaatatgc cacttaatga ccgcaacaat aaacaattat 2820
tctactagtg atcgataata aattatacta taggtcaaag ttgtgcatat tgacatgtgt 2880
taagtcaaac tgtatcgatt aatatgggaa gaaggaagta tgtaagaaaa tagcatcatg 2940
tgggatctta taatattcgt atcaatatgt atttgcaaaa tattaacttt tcacaaaatg 3000
ttttgaaagg atagagtcta ataatcaaag taataggtct attaaagtca taaatacccc 3060
taaaaaaatc ataaatacag ataatggagc aaaaatttg ggagagatta aaaattaaaa 3120
gtaattagga aagccatttc cataaggtta cttgtctttt cagagttgca cctattttta 3180
ctccactgca atggaataat actagaagca acatatataa tgtaattgga tattcttaca 3240
 
ttaatcaact aaataaaagg cctatatagt ctccaactag ttggacaatg agatgttaaa 3300
aaaaaaaaaa aaaaaaatta gttggacaat ggcatatgtt atatgttagc tatatgtcca 3360
ataaggcgac tgaaagacaa cctttaccaa attgataata ataaaaccat catcgtcaac 3420
cttcttatcc ttatgtgcct gaataatgag aacctagaca ttaccgaacg gctaacaaca 3480
agatttttac ccctaattaa gtcataacta gcgcattata tatcctttaa gtttgtcatt 3540
ctgtgatttc atttaatttt ctgtagtccc ttgccaagtc tgcctatata tatatagaag 3600
atggtgtatt gtaacttgtg acactaaatt ttcaagcatc ctcctagttt ccactttctc 3660
cttcatccac tcaacgcctt agctacgtaa gttaccaatt atgcattctc catcatacgc 3720
taatttacat tattgttaat gcttccttac atattgtttg atttaaacta ttttgtctta 3780
tcaaaattta tctgatctgg tcttattaga acttatctta tcttatatga atttaactta 3840
tattatctta tctgaactta tatcctagtt tattgtgtta tctaacatat atagttattc 3900
cgttttattc aacttattct atcgtatatc tgatataatc ttatcttatc ataacttgtt 3960
tttgttaaat aagtgaaaat aagttcaaca taacatataa tctcagtcat tcaatctgtt 4020
ttacactgtt ctttcacatt tagttgggaa ttttttaat ttttagcatt attattttcg 4080
tagacctgaa cttttcttta tctatatctc tttttattcc ctttttgttg gtaatttaca 4140
aattctagaa taagactggt gcacaacaaa gtatcgtaag ttgggggaaa tttagcagtt 4200
atagaagtga ttacttacgg ttaaaactca cctatttttt tttctaaaag cgacttcttt 4260
tttacttaca aaattacccc tataaactta aaaagtgata tttttagtct aaaagtaaaa 4320
gagtcttacc ataaattatt gtgtactcga tgctctcaag atttctatt gtttatatta 4380
ggaatatctt tgcagccctc tcgttagtaa agcttattcg aaattattac tattattcat 4440
gtccttgctt tagctaaaaa acgtcatctt ttcgttaaag ttgcaatttt cttaatccaa 4500
ttataattta catggttaac aatttcataa caaattactt actatttaac acttcctcca 4560
tttcttttaa ttacaatgct ttcacttttg catactattt attttacacc tcctccatct 4620
cttttagttg ctatacgctt tcacttttgc atactgttta tatataaatt ccaaaaagat 4680
ttattactaa cttatgcaaa taaatatcat tatgcaagat gtttagttct caatgtacac 4740
tggtgaacat ggacaaaaca ttagggaacc aagaattgaa cccccaatag attaaaaaat 4800
gaatatttac cattcattat ttattttttt gttctttgta gtccgttttt ctaaaaatta 4860
aaaacaaaat atgcaaatcg caaaaaacat ctttcacttt tcagttgcca aatttcaaaa 4920
taaacatgat tagtttaagt ttaaaacctt agtttcatga tacagctatt atcatatgac 4980
tgtaaaagtc ttaattaaac cgaaaggttg gaatttatag cgtgataccc aactgtccct 5040
ttacttctaa gagacgactt ttatatgtaa aagtatatgg gcccggacga tgctccgggt 5100
tttctctatg ctacaattta gtagtagtta aataagtcat ctcattttca tttgaaaaag 5160
ttatcttatt cattatatag attttaaaag ttttccattc atatatcatt attaatttg 5220
attgaaaaaa caataatggt atggtggagt aatggttgga tctctttatt ttatacttga 5280
ggttgtgggt tcgaatttc acgtcaacaa ttctttattt ttactatatg agaaagacgt 5340
agataaaatg ttattgaatg agggatggtg acacgaggca catatacgtt cttaggaacg 5400
ccttttaata tattagtata gatttaaaac aacatacctt taatataagc caaaatataa 5460
ttggaggttt gagactttac ttgccccagc taaaattctc ctatgttgct gaacatcaaa 5520
atattttttt tgatccatat gagcctacaa agtacaaaga aggggagggg ggatttgaac 5580
ctgtgaccta tcgttcacat cacctcaatc ttaaccacta ggccaagaca tccttggtta 5640
ctgaacatca aaatataatt ggagggtaat tgttactatc taatagatta ttaaatatat 5700
taaagatcaa aaattataca ttcgaaagca tgaaagttaa acacgtaaca aacgaattaa 5760
gtatacgctg tattattttc atattttatg ctatgataca gatgcattag tgtgacaaga 5820
aaaccatgg 5829
<212> Type : DNA
<211> Length : 5829
   SequenceName : 4
   SequenceDescription :

### Feature

Sequence: 4:
   <221> FeatureKey : Translationstart-ATG
   <222> LocationFrom : 5826
   <222> LocationTo : 5828
      Other Information :
      CDSJoin : No

### Sequence

<213> OrganismName : Beta vulgaris
<400> PreSequencestring : 
aagcttaaat gtgtaagcgg atttatgtta caagttattt gttagagata gatctaatta 60
tatgtactct tcttagattg attccatcaa atttcatcaa ccttagcatt tgccttcctg 120
tagcttgaag actggcgaca actgcttttg aagaaagaag aatgcggagt attgcttttg 180
cccacacaca tgctcctaca actccaaaat tgccagctct tatgcctatt ttgagaacca 240
tgttatcatg caaatttgta tataatcaga tggtgtatgc acttttttgg acaagctcaa 300
ctaacagagc aacctaatgt aggaaggaaa caaatttaca agtattaaca tcttgccggc 360
 
attgctctaa ccaggaacat tagtcttata gtcttaaagt tattataggt taatgtgtat 420
tagatatcta cgtaaccgca tccaaattgc gcaaattcta caatatccgt aacacaacaa 480
acatacatct acactttgtt tcatagcgtg cgaaaccact ttactacttt gaggcaccta 540
aacgactaca aatagcacca ttctactatt tcggagaatc atacaatgcc tcaaaaacca 600
tgtagatgta atcaatttta gtacgcacac atatcctccg tgaattgacc actgcaattc 660
aaacaaatag tgtgcttacc acctatttga aatcaattac aaacaaatag caccgtactt 720
attataccta acgaattacg aataacaatt acgctatttt ggggtgccgc gcgcgtaaac 780
aactaatctc attcaaaaag gtcaaattag agacattgtg gttacgtact gcgcgccacc 840
tacccctttc tcgggccttc atgacgtgtc ctatcacaat cttctgttga gataatcttt 900
ccaaccgcct aaccttttct tatcttaatt tttcttttcc ccttttaccg ccaaattaag 960
ccacaaaccc ttgtacacaa ctaaatgcac gcacatccgt ctgatcatct atcacccatg 1020
caatctcagc cgtttattat ttcttttttg tcccctatat atataataat tcctccttta 1080
ataaatctta tcattcattc attgaataca tccatgg 1117
<212> Type : DNA
<211> Length : 1117
   SequenceName : 5
   SequenceDescription :

### Feature

Sequence: 5:
   <221> FeatureKey : Translationstart-ATG
   <222> LocationFrom : 1114
   <222> LocationTo : 1116
      Other Information :
      CDSJoin : No

### Sequence

<213> organismName : Künstliche Sequenz
<400> Presequencestring :
   gtgcaaggat tctggcaccc gtcggtgg 28
<212> Type : DNA
<211> Length : 28
   SequenceName : 6
   SequenceDescription :

### Sequence

<213> organismName : Künstliche Sequenz
<400> PreSequenceString :
   gtatgggccg cggcagatcc aggtagcg 28
<212> Type : DNA
<211> Length : 28
   SequenceName : 7
   SequenceDescription :

### Sequence

<213> organismName : Künstliche Sequenz
<400> PreSequenceString :
   atgtttaagt acgacagtgt tcacg 25
<212> Type : DNA
<211> Length : 25
   SequenceName : 8
   SequenceDescription :

### Sequence

<213> OrganismName : Künstliche Sequenz
<400> Presequencestring :
   atgtgaaggt ctgacttgta ttcgt 25
<212> Type : DNA
<211> Length : 25
   SequenceName : 9
   SequenceDescription :

### Sequence

<213> organismName : Künstliche Sequenz
<400> Presequencestring :
   gtggagaggc tattcggta 19
<212> Type : DNA
<211> Length : 19
   SequenceName : 10
   SequenceDescription :

### Sequence

<213> organismName : Künstliche Sequenz
<400> PreSequenceString :
   ccaccatgat attcggcaag 20
<212> Type : DNA
<211> Length : 20
   SequenceName : 11
   SequenceDescription :

## Patentansprüche

1. Promotor mit einer Nukleotidsequenz gemäß SEQ ID NO: 4 oder einer zu der Nukleotidsequenz gemäß SEQ ID NO: 4 komplementären Nukleotidsequenz oder einer Nukleotidsequenz, die mit der Nukleotidsequenz gemäß SEQ ID NO: 4 oder einer zu der Nukleotidsequenz gemäß SEQ ID NO: 4 komplementären Nukleotidsequenz unter stringenten Bedingungen in 4 x SSC bei 65 °C und anschließend mehrfachem Waschen in 0,1 x SSC bei 65 °C für insgesamt eine Stunde hybridisiert.

2. Vektor oder mobiles genetisches Element, enthaltend einen Promotor nach Anspruch 1.

3. Eukaryotische oder prokaryotische Wirtszelle, die mit einem Promotor nach Anspruch 1 transformiert wurde und diesen transformierten Promotor enthält.

4. Pflanze oder deren Teile, die mit einem Promotor nach Anspruch 1 transformiert wurden und diesen transformierten Promotor enthalten.

5. Pflanze nach Anspruch 4, **dadurch gekennzeichnet, dass** die Pflanze *Beta vulgaris* ist.

6. Samen von Pflanzen nach Anspruch 4 oder 5, wobei die Samen einen Promotor nach Anspruch 1 enthalten, mit dem die Pflanzen transformiert wurden.

7. Verwendung eines Promotors nach Anspruch 1 zur Herstellung einer transgenen Pflanze mit einer oder mehreren der folgenden Eigenschaften:
a) Verbesserter Kohlenhydratmetabolismus in Speicherorganen nach der Ernte
b) Verbesserter Stickstoffmetabolismus in Speicherorganen nach der Ernte
c) Verbesserte Trockenstressresistenz und verbesserter Wasserstatus in Speicherorganen nach der Ernte
d) Verbesserte Kälte- und Frosttoleranz von Speicherorganen nach der Ernte
e) Erhöhte Resistenz/Toleranz von Speicherorganen gegenüber Pathogenen nach der Ernte
f) Verbesserter Sekundärstoffwechsel in Speicherorganen nach der Ernte

## Claims

1. A promoter comprising a nucleotide sequence according to SEQ ID NO: 4 or a nucleotide sequence complementary to the nucleotide sequence according to SEQ ID NO: 4 or a nucleotide sequence that hybridizes with the nucleotide sequence according to SEQ ID NO: 4 or with a nucleotide sequence complementary to the nucleotide sequence according to SEQ ID NO: 4 under stringent conditions in 4 x SSC at 65 °C and followed by multiple washing in 0.1 x SSC at 65 °C.

2. A vector or mobile genetic element including a promoter according to claim 1,

3. A eukaryotic or prokaryotic host cell transformed with a promoter according to claim 1 and Including said transformed promoter.

4. A plant or parts thereof transformed with a promoter according to claim 1 and including said transformed promoter.

5. The plant according to claim 4, **characterized in that** the plant is *Beta vulgaris.*

6. Seeds from plants according to claim 4 or 5, whereby the seeds include a promoter according to claim 1, with which the plants had been transformed.

7. Use of a promoter according to claim 1 for producing a transgenic plant with one or more of the following properties:
a) improved carbohydrate metabolism in the storage organs after harvest
b) improved nitrogen metabolism in the storage organs after harvest
c) improved drought stress resistance and improved water status in the storage organs after harvest
d) improved cold and frost tolerance of the storage organs after harvest
e) increased resistance/tolerance of the storage organs against pathogens after harvest
f) improved secondary metabolism in the storage organs after harvest.

## Revendications

1. Promoteur ayant une séquence de nucléotides selon SEQ ID N : 4 ou une séquence de nucléotides complémentaire à la séquence de nucléotides selon SEQ ID N° : 4 ou une séquence de nucléotides qui s'hybride, dans des conditions stringentes, avec la séquence de nucléotides selon SEQ ID N° : 4 ou avec une séquence de nucléotides complémentaire à la séquence de nucléotides selon SEQ ID N° : 4, ladite hybridation étant réalisée dans 4 x SSC à 65 °C et étant suivie de plusieurs lavages dans 0,1 x SSC à 65 °C, pendant une durée totale d'une heure.

2. Vecteur ou élément génétique mobile, contenant un promoteur selon la revendication 1.

3. Cellule hôte d'un eucaryote ou d'un procaryote laquelle a été transformée avec un promoteur selon la revendication 1 et contient ledit promoteur faisant l'objet de la transformation.

4. Plante ou parties de celle-ci ayant été transformée(s) avec un promoteur selon la revendication 1 et contenant ledit promoteur faisant l'objet de la transformation.

5. Plante selon la revendication 4, **caractérisée en ce que** ladite plante est *Beta vulgaris.*

6. Graines de plantes selon les revendications 4 ou 5, lesdites graines contenant un promoteur selon la revendication 1 avec lequel lesdites plantes ont été transformées.

7. Utilisation d'un promoteur selon la revendication 1 pour obtenir une plante transgénique ayant une ou plusieurs des propriétés suivantes :
a) un métabolisme amélioré des glucides dans les organes de réserve après la récolte
b) un métabolisme amélioré de l'azote dans les organes de réserve après la récolte
c) une résistance améliorée au stress hydrique et un statut hydrique amélioré dans les organes de réserve après la récolte
d) une résistance améliorée au froid et au gel en ce qui concerne les organes de réserve après la récolte
e) une résistance / tolérance accrue vis-à-vis de pathogènes en ce qui concerne les organes de réserve après la récolte
f) un métabolisme secondaire amélioré dans les organes de réserve après la récolte
